# FASCICULE DE BREVET EUROPEEN

(11) **EP 0 517 990 B1**
(45) Date de publication et mention de la délivrance du brevet: **10.03.1999**
(21) Numéro de dépôt: 91810445.6
(22) Date de dépôt: 12.06.1991
(51) Int. Cl.: A63B 23/18

(54) **Dispositif ergométrique**
Ergometrische Vorrichtung
Ergometric device

(43) Date de publication de la demande: 16.12.1992
(73) Titulaire: TRADOTEC S.A., 1214 Vernier (CH)
(72) Inventeur: Tkatchouk, Elena N., 103473 Moscow (SU); Tsyganova, Tatiana N., 105077 Moscow (SU); Murashov, Mikhail V., 127562 Moscow (SU); Staebler, Regula, CH-1214 Vernier (CH)
(74) Mandataire: Charbonnier, Georges R.

(56) Documents cités:
- WO-A-89/07465
- DE-A- 3 121 868
- US-A- 4 634 117
- WORLD PATENTS INDEX LATEST Section PQ, Week 9037, Derwent Publications Ltd., London, GB; Class P36, AN 90-275555 & CN-A-1 037 661 (G JIANG) 6 Décembre 1989

## Description

L'invention a pour objet un dispositif ergométrique comprenant un appareil ergométrique, un générateur de mélanges gazeux hypoxiques, un masque, un tuyau souple reliant le générateur au masque, le tout de manière que l'utilisateur de l'appareil ergométrique puisse inhaler le mélange gazeux hypoxique produit par le générateur.

Le brevet US No 3 871 648 décrit une bicyclette ergométrique fixe imitant des charges routières, équipée de moyens de réglage de l'intensité de ces charges.

Le brevet US No 4 079 931 décrit un appareil ergométrique qui permet d'effectuer des exercices physiques répétitifs, comportant un siége et un guidon, également équipé de moyens de réglage de la charge à vaincre par son utilisateur.

Le brevet US No 4 441 705 décrit également un appareil ergométrique dans lequel l'utilisateur entraine une roue dotée de moyens de freinage réglables.

L'inconvénient de ces appareils réside dans le fait qu'il est impossible d'empêcher les surcharges et de fournir des charges-limites sécuritaires, notamment dans les cas de personnes atteintes de pathologies de l'organisme du type cardio-vasculaire, pulmonaire ou autres.

Par ailleurs, la demande de brevet publiée DE 31 21 868 décrit un appareil respiratoire produisant un mélange gazeux hypoxique.

Le dispositif ergométrique selon la présente invention permet à l'utilisateur de l'appareil ergométrique de dépenser de l'énergie physique en quantité mesurée et contrôlée, dans un intervalle de temps réglable, en réduisant les risques consécutifs aux dépassement des charges-limites programmées et les rendre ainsi plus sécuritaires.

A cet effet, ce dispositif est caractérisé par le fait qu'il comprend des moyens permettant de programmer, en temps et en débit, le mélange gazeux hypoxique alimentant le masque.

L'inhalation du mélange hypoxique peut être continue, discontinue ou alternée avec de l'air.

Cette inhalation, effectuée de manière contrôlée, a pour effet, comme de nombreux tests l'ont démontré, de réduire les risques consécutifs aux surcharges et de rendre par conséquent plus sécuritaires, les charges limites, aussi bien lors d'entrainement physiques de personnes en bonne santé, de sportifs par exemple, que lors d'exercices effectués par des sujets dont l'organisme présente des pathologies d'un des types susdits.

L'inhalation continue, discontinue du mélange hypoxique, ou encore alternée avec de l'air, programmée de façon bien déterminée dans chaque cas, présente les avantages suivants :
- Amélioration de la résistance de l'organisme aux surcharges brèves ou excessives,
- Adaptation de l'organisme aux stress associés à ces surcharges,
- Accroissement de l'oxyphorèse sanguine

Ces différents effets favorisent une meilleure stabilité du métabolisme énergétique ce qui permet à l'oxygène consommé par l'utilisateur d'être exploité de façon plus efficace en particulier au niveau de ses organes vitaux.

On réglera évidemment le dosage oxygène/azote du mélange gazeux hypoxique en fonction des charges physiques à vaincre par le sujet et à l'adaptation de son organisme à ce mélange.

Les expériences ont montré que cette méthode est non seulement valable pour les personnes en bonne santé, par exemple pour les sportifs, mais a également des effets remarquables, du point de vue thérapeutique, sur des personnes affaiblies par des pathologies cardio-vasculaires, pulmonaires, gastro-intestinales, névralgiques, dermatologiques et gynécologiques.

Dans ces cas, les charges physiques mesurées et contrôlées, et l'inhalation d'un mélange gazeux hypoxique tonifient l'organisme, assurent à long terme une rémission des pathologies et augmentent la résistance de l'organisme.

Le programme d'ihhalation du mélange hypoxique pourra par example prévoir des périodes de 2 à 15 minutes entrecoupées d'intervalles de 2 à 5 minutes de respiration d'air de façon à rétablir les fonctions respiratoires et à augmenter le niveau d'oxygène dans les poumons. On pourra accroître gra⁻ duellement la durée d'inhalation du mélange gazeux hypoxique et celle des charges physiques de 15 à 60 minutes par jour pour un traitement complet de 10 à 30 jours.

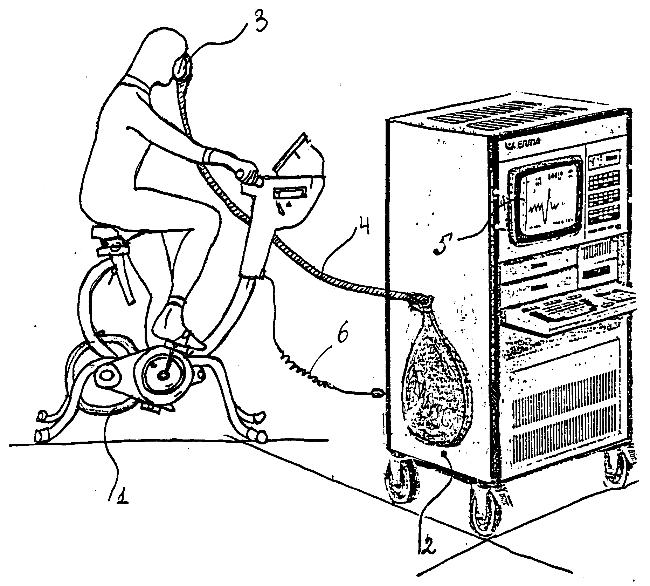

La figure unique du dessin ci-annexé représente schématiquement et à titre d'exemple une forme d'exécution de l'objet de l'invention.

Le dispositif représenté comprend une bicyclette d'exercice ergométrique fixe 1, dotée de moyens de réglage de la charge, un générateur de mélange gazeux hypoxique 2 et un masque 3, ajustable sur le visage de l'utilisateur et relié au générateur 1 par un tuyau souple 4.

Le générateur 1, qui peut fournir un mélange gazeux hypoxique constitué en volume de 9 à 16% d'oxygène et de 84 à 91% d'azote, comprend, monté dans un chariot, un compresseur membranaire, une unité de séparation gazeuse ( y compris une membrane réalisée en poly-4-méthyl-penthène-1 ), une soupape régulatrice, un filtre pour retenir les éventuelles impuretés, un humidificateur de mélange gazeux, et, sur la face frontale de la carrosserie du chariot, des moyens permettant de programmer le traitement et de régler les composants du mélange gazeux hypoxique, ainsi qu'un écran 5 sur lequel sont visualisés l'électrocardiogramme de l'utilisateur, son pouls et sa pression sanguine, les données correspondantes étant transmises par l'intermédiaire d'un capteur fixé sur l'utilisateur et d'une liaison souple 6.

Le mélange gazeux hypoxique fournit par le générateur 2 arrive sur le visage de l'utilisateur à travers le masque 3 et le tuyau 4.

Assis sur la selle de la bicyclette 1, l'utilisateur dépense de l'énergie en pédalant pendant qu'il inhale de façon continue ou discontinue, ou alternativement avec de l'air, selon un programme déterminé, le mélange hypoxique.

Une personne en bonne santé, par exemple un sportif, utilisant le dispositif pour son entrainement physique ou pour se maintenir en forme, pourra pédaler à des vitesses de l'ordre de 60 révolutions/minute avec des charges déterminées basées sur des limites fonction de son âge, de son poids et de sa condition physique.

La puissance de ces charges pourra, par exemple, passer de 10 watts au début, à 15 watts dès le 10 ème jour, et à 25 watts à partir du 20 ème jour.

En pédalant, l'utilisateur inhalera, par exemple, durant 15 à 45 minutes, un mélange hypoxique composé, en volume, de 10% d'oxygène et de 90% d'azote. Cette inhalation sera avantageusement de durées de 4 à 7 minutes entrecoupées d'intervalles de respiration d'air pur de 2 à 3 minutes.

La durée d'une session d'entrainement s'étendra de préférence pour un sportif sur une période de 20 jours environ.

La durée d'inhalation continue du mélange hypoxique sera progressivement augmentée de 15 à 45 minutes par séance.

Lors de chaque séance d'entrainement le pouls et la pression sanguine du sportif devront demeurer en dessous des limites prévues mais une surcharge de courte durée, même relativement importante, n'aura aucune conséquence sur son organisme.

Au cours d'un tel entrainement le sportif se sent bien, sans signe de stress excessif il dort détendu et son humeur est bonne.

L'adaptation de l'organisme de l'utilisateur aux charges programmées est visualisée sur l'écran 5.

Avec le même dispositif, on pourra soigner une personne présentant au niveau de son organisme, une pathologie cardio-vasculaire, une maladie ischémique du coeur,en réglant la charge à une puissance de 10 watts, et la vitesse du pédalier à 40 révolutions par minute

La durée de chaque séance sera fixée à 10 à 15 minutes et la session réduite à 20 jours par exemple.

Dans ce cas, on programmera le dispositif de façon que le patient inhale le mélange gazeux hypoxique durant des périodes de 3 à 5 minutes entrecoupées d'intervalles de 2 à 3 minutes de respiration d'air.

La durée totale d'inhalation du mélange gazeux hypoxique sera de l'ordre de 15 minutes le premier jour puis augmentée progressivement jusqu'à 60 minutes le dernier jour de traitement.

Comme dans le premier cas décrit, on suivra l'évolution du pouls et de la pression sanguine du patient et son électro-cardiogramme sur l'écran 5.

## Revendications

1. Dispositif ergométrique comprenant un appareil ergométrique (1), un générateur de mélanges gazeux hypoxiques (2), un masque (3), un tuyau souple (4) reliant le générateur (2) au masque (3), le tout de manière que l'utilisateur de l'appareil ergométrique (1) puisse inhaler, durant ses exercices, le mélange gazeux produit par le générateur (2), caractérisé par le fait qu'il comprend des moyens permettant de programmer, en temps et en débit, le mélange gazeux hypoxique alimentant le masque (3).

2. Dispositif selon la revendication 1, caractérisé par le fait qu'il comprend des moyens de réglage des composants du mélange gazeux hypoxique.

## Claims

1. An ergometric unit comprising an ergometric device (1), a generator of hypoxic gas mixtures (2), a mask (3) and a flexible tube (4) linking the generator (2) to the mask (3), so that the user can inhale, while exercising, the gas mixture produced by the generator (2), characterised in that the ergonomic unit includes the facility to program, by time and by output, the hypoxic gas mixture feeding into the mask (3).

2. Device as claimed in claim 1, characterised by means to adjust the composition of the hypoxic gas mixture.

## Patentansprüche

1. Ergometrische Vorrichtung, umfassend einen ergometrischen Apparat (1), einen Generator von hypoxischen Gasgemischen (2) und eine Maske (3), welche so angeordnet sind, daß der Benutzer der ergometrischen Vorrichtung (1) während seiner Übungen eine Gasmischung einatmen kann, die vom Generator (2) geliefert wird, dadurch gekennzeichnet, daß er die Mittel umfaßt, die es erlauben, nach Zeit und Leistung das hypoxische Gasgemisch, welches die Maske (3) versorgt, zu programmieren.

2. Vorrichtung nach Anspruch 1, dadurch gekennzeichnet, daß sie Mittel für die Einstellung der Bestandteile des hypoxischen Gasgemisches umfaßt.
